Europäisches Patentamt

(19) European Patent Office

Office européen des brevets

(11) Publication number: **0 269 728 B1**

(12) # EUROPEAN PATENT SPECIFICATION

(45) Date of publication of patent specification: **11.12.91 Bulletin 91/50**

(21) Application number: **87904861.9**

(22) Date of filing: **20.05.87**

(86) International application number: **PCT/EP87/00264**

(87) International publication number: **WO 87/07302 03.12.87 Gazette 87/27**

(51) Int. Cl.$^5$: **C12P 21/08, C12N 5/12, G01N 33/577, // (C12P21/00, C12R1:91)**

(54) **MONOCLONAL ANTIBODIES TO HUMAN LYMPHOCYTE IgE RECEPTORS, HYBRIDOMAS PRODUCING SUCH ANTIBODIES, AND KITS FOR USING SUCH ANTIBODIES.**

(30) Priority: **27.05.86 FR 8607583**

(43) Date of publication of application: **08.06.88 Bulletin 88/23**

(45) Publication of the grant of the patent: **11.12.91 Bulletin 91/50**

(84) Designated Contracting States: **AT BE CH DE FR GB IT LI LU NL SE**

(56) References cited:
Chemical abstracts, vol. 104, n 15, April 1986, (Columbus, Ohio, US), page 556, abstract 128114y & JP A 60255734 (Nichirei Corp.), 17 December 1985
Biological abstracts, vol. 29, n 17368, 1985 (Philadelphia, PA, US) T. Nakajima et al "Identification of the membrane molecules from RPMI-8866 cells reacting with amonoclonal antibody specific to FC-epsilon-R" & Journal of Allergy and Clinical Immunology (US), 1985, vol. 75, n 1, part 2, p. 139
Biological abstracts, vol. 29 n 15591, 1985 (Philadelphia, PA. US) M. Sarfati et al "Production of antihuman FC-epsilon-R antibody upon immunization with monoclonal anti-human immunoglobulin E antibody" & Federation Proceedings (US), 1985, vol. 44, n 3, p. 784
Biological abstracts, vol. 80 n 6, 1985 (Philadelphia, PA, US) T. Nagai et al "T and B lymphocytes with immunoglobulin E Fc receptors in patients with nonallergic hyperimmunoglobulinemia E : Demonstration using a monoclonal antibody against immunoglobulin E fc receptor-associated antigen", see page AB-456, abstract 49916 & Clin. Immunol. Immunopathol 35(3), pp. 261-275, 1985

(73) Proprietor: **SCHERING-PLOUGH**
**92, rue Baudin**
**F-92307 Levallois-Perret (FR)**

(72) Inventor: **BONNEFOY, Jean, Yves**
**Les Terrasses de Parsonges Bâtiment E**
**F-69570 Dardilly (FR)**
Inventor: **WIJDENES, John**
**8, rue Jean Perréal**
**F-69008 Lyon (FR)**
Inventor: **PERONNE, Catherine**
**70, rue de la Charité**
**F-69002 Lyon (FR)**
Inventor: **AUBRY-LACHAINAYE, Jean-Pierre**
**4, rue des Fraisiers**
**F-69630 Chaponost (FR)**
Inventor: **BANCHEREAU, Jacques**
**25, avenue Paul Santy**
**F-69130 Ecully (FR)**

(74) Representative: **Durand, Yves Armand Louis et al**
**Cabinet Z. Weinstein 20, Avenue de Friedland**
**F-75008 Paris (FR)**

(56) References cited :

The Journal of Immunology, vol. 30, n 4, April 1983, The American Association of Immunologists (US), D.S. Finbloom et al "Isolation of croos-linked IgE-receptor complexes from rat macrophages", pages 1489-1491

The Journal of Immunology, vol. 129, n 2, August 1982, The American Association of Immunologists (US), F.M. Melewicz et al "Comparison of the Fc receptors for IgE on human lymphocytes and monocytes", pages 563-569

The Journal of Immunology, vol. 132, n 2, February 1984, The American Association of Immunologists (US), D.H. Conrad et al "The murine lymphocyte receptor for IgE. I. Isolation and characterization of the murine B cell FcE receptor and comparison with FcE receptors from rat and human", pages 796-803

The Journal of Immunology, vol. 137, n 4, 15 August 1986, The American Association of Immunologists (US), N. Noro et al "Monoclonal antibody (H107) inhibiting IgE binding to FcER (+) lymphocytes", pages 1258-1263

The Journal of Immunology, vol. 137, n 4, 15 August 1986, The American Association of Immunologists (US), M. Suemura et al "Monoclonal anti-fcE receptor antibodies with different specificities and studies on the expression of FcE receptors on human B and T cells", pages 1214-1220

## Description

## I. INTRODUCTION

This invention relates to novel monoclonal antibodies to the human lymphocyte IgE receptors, that is, to monoclonal antibodies directed at the receptor for the Fc fragment of human IgE which is present in lymphoid cells ($Fc_\varepsilon R_{II}$), to hybridomas producing such antibodies, to kits for using such antibodies, e.g. in assaying or detecting these receptors on cells, and to related soluble molecular entities of the IgE binding factor type (=IgE BF).

It is well known in the art that it is possible to obtain a cell line which is able to produce a homogenous, i.e. monoclonal, antibody. The basic technique (Kohler and Milstein, Nature 256, 1975) comprises the fusion of mouse myeloma cells with spleen cells to form hybridoma cells and selection from these of clones capable of producing the desired antibody. This general procedure has also been described in U.S. Patents 4,364,932, 4,364,934, 4,364,935, 4,364,937 and 4,361,550.

Although the general method has been known for some years, the preparation and selection of each suitable hybridoma presents its own special difficulties. There is indeed no certainty that a suitable hybridoma will be found and, equally, there is no certainty that the hybridoma will produce an antibody having the desired properties.

Monoclonal antibodies have a variety of uses, in particular for the isolation and purification of the proteins to which they are specific or for assaying them e.g. in a diagnostic kit; see for example PCT published applications WO81/02899 and WO82/01773.

The receptor for the Fc fragment of Immunoglobulin E ($Fc_\varepsilon R_{II}$) is a glycoprotein expressed on the surface of certain human cells. It has the capacity of binding IgE and therefore can be considered an IgE binding factor. Suppressive IgE BFs would be of considerable interest in the therapy of diseases linked to elevated IgE levels, such as allergic rhinitis, atopic dermatitis and asthma. IgE potentiating factors, on the other hand, would have utility in the treatment of conditions where increased IgE levels might be required (e.g., elimination of parasites). Assays for such factors exist, but the availability of monoclonal antibodies (Mabs) would greatly help in designing highly sensitive and specific immunochemical assays.

It is therefore an object of the present invention to provide the following:

Monoclonal antibodies inhibiting binding of soluble IgE to human lymphocytes;

Monoclonal antibodies to the human lymphocyte IgE receptors;

Methods for their preparation;

Hybridomas producing them;

Assays using them (for detecting them on human cells);

Kits using them for detection of soluble forms (e.g., in body fluids or culture supernatants).

The invention therefore provides monoclonal antibodies inhibiting binding of soluble IgE to human lymphocytes. The invention further provides monoclonal antibodies to the human lymphocyte IgE receptors. These monoclonal antibodies are preferably of the immunoglobulin subclass $IgC_1$. The invention further provides monoclonal hybridomas capable of producing these monoclonal antibodies.

According to the invention, monoclonal antibodies inhibit the binding of soluble IgE to human lymphocytes, and are identical or similar to monoclonal antibodies produced from hybridomas obtained according to the following successive steps :

a) The culture of hybridomas is obtained, using several separate containers, by fusion of mice myeloma cells with spleen cells obtained from mice immunized by injections of cells which strongly express IgE receptor, e.g. RPMI 8866, preferably in a medium eliminating unfused myeloma cells and unfused spleen cells, e.g. in a HA medium;

b) the supernatants of the hybridoma cells of each container are tested for the presence of antibodies inhibiting the binding of IgE to the said cells;

c) hybridomas producing antibodies inhibiting the binding of IgE to the said cells are selected;

d) these selected hybridomas are cloned, preferably by the limiting dilution technique, these clones being chosen so as to obtain the desired monoclonal antibodies by culturing the hybridomas.

These monoclonal antibodies are preferably of the immunoglobulin subclass $IgG_1$.

The aforesaid antibodies are the monoclonal antibody Mab 25 produced by the hybridoma 9P25 A 3/13 deposited at the Institut Pasteur under N° I-548, and the monoclonal antibody Mab 135 produced by the hybridoma 9P 135 D 6/5 deposited at the Institut Pasteur under N° I-549.

The assays for soluble IgE BFs can be of two types, a competition assay or a double sandwich assay (which requires two Mabs binding to different epitopes of the relevant antigen).

In the competition assay, a human IgE binding factor (IgE BF) labelled e.g. with an enzyme (for example E. coli β-galactosidase) or a radioisotope competes with a non-labelled one (present in the sample to be assayed or a standard) for binding to a Mab linked to a solid support. This requires only one Mab.

In order to produce Mabs specific for human IgE binding factors, mice were immunized with cells expressing on their surface such molecules at high density. Alternatively, mice could be immunized with soluble IgE binding factors.

## II. ASSAY FOR HUMAN $Fc_\varepsilon R_{II}$ AND CHOICE OF THE CELL LINE EXPRESSING $Fc_\varepsilon R_{II}$ AT HIGH DENSITY.

### INTRODUCTION

The human cell line RPMI 8866 is known to express $Fc\varepsilon R_{II}$ at relatively high density. An assay has been established which permits detection of $Fc_\varepsilon R_{II}$. Briefly, cells to be assayed are successively incubated with IgE, a monoclonal (or polyclonal)anti-IgE antibody, and fluorescent microspheres coupled with anti-mouse (or rabbit) immunoglobulin antibody. The percentage of cells binding to the fluorescent microspheres (corresponding to the percentage of cells expressing $Fc_\varepsilon R_{II}$) is determined by flow cytometry. Details of this are given in a separate section.

This assay was found suitable for the detection of human IgE BF: Preincubation of IgE with IgE BF results in a decreased binding of IgE to its receptor so that a decreased percentage of cells bind to the fluorescent microspheres. This work demonstrated that RPMI 8866 was the best cell line for immunizing mice since it carried much $Fc_\varepsilon R_{II}$.

Mabs against $Fc_\varepsilon R_{II}$ can be screened for their ability to inhibit the binding of IgE to RPMI 8866 cells. Cells are first incubated with the Mab to be assayed and then IgE is added. The other steps are carried out as described above.

## FLOW CYTOMETRIC METHOD FOR DETECTION OF $Fc_\varepsilon$ RECEPTORS AND IgE BINDING FACTORS USING FLUORESCENT MICROSPHERES

### MATERIALS AND METHODS

#### Antibodies and proteins:

Human myelomatous IgE was purified by ion exchange chromatography (DEAE 52) using a NaCl gradient (0.01M - 0.3M, pH 7.2). According to SDS-PAGE and silver staining, the IgE was pure and uncontaminated by other proteins.

To detect IgE, a monoclonal anti-IgE (Hybritech Inc., La Jolla, CA) and a polyclonal anti-IgE were used. The polyclonal antiserum was prepared by immunizing rabbits with purified IgE PS, and the antiserum was rendered monospecific for IgE by passage over IgG, IgA and IgM immunoabsorbant columns (Affigel-10 Biorad, Richmond, CA).

A rabbit anti-mouse Ig antiserum and a goat anti-rabbit Ig antiserum have been prepared in our laboratory. Their IgG fractions were isolated by column chromatograpy using Trisacryl GF 05 and DEAE Trisacryl (Industrie Biologique Francaise, Genevilliers, France). Human IgG, IgA and IgM were purified from myeloma or Waldenstrom sera using ion exchange chromatography according to standard procedures.

#### CELLS:

WIL2WT (an EBV (= Epstein-Barr Virus) lymphoblastoid cell line), Daudi (a Burkitt lymphoma), Molt 4 (a T leukemia), HL60 (a promyelomonocytic leukemia) and U937 (a promonocytic cell line) were obtained from the ATCC (Americain Type Culture Collection; Rockville, Maryland). RPMI 8866 (an EBV lymphoblastoid cell line) was kindly provided by Dr. K. Ishizaka. UD38 is an EBV lymphoblastoid cell line which has been established in our laboratory by Dr. F. Rousset after infection of normal peripheral blood B cells with EBV. All cells were cultured in RPMI 1640 (Flow, Irvine, Scotland) supplemented with 10% heat inactivated fetal calf serum (Flow), 2mM glutamine (Flow), 100 U per ml penicillin and 100 µg per ml streptomycin (Flow).

#### Coupling of proteins to microspheres

0.1 mg of the protein to be coupled is dissolved in 1 ml of a solution of EDAC (N-(3-dimethylaminopropyl)-N'-ethyl-carbodiimide hydrochloride) (0.1 mg per ml distilled water).

100 µl sonicated (1 min in a Bransonic 321) microspheres (Fluoresbrite carboxylate diameter 0.57 µ, Polyscience, Warrington, PA, ref 15700) are added. The suspension is incubated overnight at 4°C on a rocking table. After this incubation, the microspheres are centrifuged (10000 g, 5 min) and washed three times with PBS-BSA (phosphate-buffered saline containing 1% bovine serum albumin). The microspheres are finally kept at 4°C in PBS-BSA supplemented with 15 mM sodium azide. The suspension is sonicated for 1 minute before each experiment.

## Staining protocol

Cells are adjusted to $10^7$/ml in PBS-BSA. $5 \times 10^5$ cells (50 µl) are distributed per well of 96 conical wells microtiter plates (Flow, Reference number: 7632105.)

The cells are first incubated 45 min with monomeric IgE (0.5 to 2 µg per $5 \times 10^5$ cells). Cells are then washed with PBS-BSA. A polyclonal anti-IgE antibody appropriately diluted (e.g., 1/2000) is added. After 45 min, the cells are washed with PBS-BSA. The anti-rabbit Ig microspheres (dilution 1/50) are added.

This cell suspension is layered on top of 3 ml heat-inactivated and filtered FCS in conical tubes (Falcon Ref 2099). After centrifugation at 100 X g for 15 min, the supernatant is slowly aspirated and PBS-BSA (150 µl/well) is added to the cell samples which, after resuspension, are analysed by flow cytometry. All steps are carried out at 4°C.

## Fluorescence analysis

Fluorescence analysis was performed with a flow cytometer, FACS 440 (Becton Dickinson, Sunnyvale, CA), equipped with a 5 W argon laser running at 488 nm, 0.5 W. Fluorescence parameters were collected using the built-in logarithmic amplifier after gating on the combination of forward light scatter (FLS) and perpendicular light scatter (PLS) which was used to distinguish viable cells from non-viable ones.

Data were stored in list mode and analysed with a PDP 11/23.

## III. IMMUNIZATION OF MICE AND PRODUCTION OF HYBRIDOMAS SECRETING Fc$_\epsilon$R$_{II}$-SPECIFIC MABS

1. Mice are immunized three times with $50 \times 10^6$ RPMI 8866 cells injected intraperitoneally in phosphate-buffered saline (PBS). The type of mouse used should not be critical but good results are achieved with BALB/c females. The number of injections and the quantity of cells administered must be such that useful quantities of suitably primed splenocytes are produced. The serum of properly immunized mice inhibits the binding of IgE to RPMI 8866 cells.

2. The immunized mice are killed, their spleens are removed and spleen suspensions are prepared. This procedure follows well-known techniques.

3. The spleen cells are fused with mouse myeloma cells. The technique for fusing myeloma cells with spleen cells is well known. Most preferably the fusion is achieved by warming a mixture of the two cell types with an appropriate fusion promoter, e.g. polyethyleneglycol (PEG) having an average molecular weight from about 1000 to about 4000 (PEG1000). Several mouse myeloma cell lines are known and easily available. Preferred are cell lines which are HGPRT-deficient (HGPRT = Hypoxanthine Guanosyl Phosphoribosyl Transferase) and accordingly will not survive in HA (culture medium comprising hypoxanthine and azaserine). Preferably the myeloma cell line used should be of the non-secreting type in that it does not itself produce any antibody. A suitable cell line for the purpose of this invention is the so-called NSI cell line. These cells were derived from P3/X63-A8 myeloma cells by Kohler and Milstein.

4. The fused spleen cells are cultured in several separate containers (e.g. in 24-well plates) according to standard procedures. The cell cultures obtained in step 3 are mixtures of fused spleen cells (hybridoma cells), unfused spleen cells and unfused myeloma cells. Preferably the cultivation is carried out in a medium which will eliminate the unfused myeloma cell line, e.g. in an HA medium. Those unfused spleen cells which are non-malignant will normally die after a short period of time, whereas the fused cells, which are HGPRT$^+$, can grow in HA medium.

5. The supernatants of the hybridoma cells in each container are tested for the presence of anti-Fc$_\epsilon$R$_{II}$ antibodies. The hybridomas are screened for their ability to inhibit the binding of IgE to RPMI 8866 cells. This test may conveniently be carried out by means of the bead assay described above.

6. Hybridomas producing the desired antibodies are selected and then are cloned preferably by the limiting dilution technique.

7. The desired antibodies are produced by means of the selected hybridomas. This production may be achieved in vitro by culturing the hybridoma in a suitable medium followed by isolation of the antibody, but this method may not yield sufficient quantities. For producing larger quantities of the antibody, an in vivo

5

method is preferably used. The hybridoma is injected back into mice where it will cause production of ascites fluid containing substantial quantities of the desired antibody, which is then isolated according to standard procedures.

## IV. CHARACTERISTICS OF THE PRODUCED HYBRIDOMAS

### 1. Inhibition of IgE binding

Two hybridomas, 9P25 and 9P135 D6/5, which gave two clones 25 and 135 D6/5 respectively, have been selected since they produce Mabs (called Mab 25 and Mab 135 respectively) that inhibit the binding of IgE to RPMI 8866 cells; see Table I:

## TABLE I

```
Inhibition of IgE Binding by Mab 25 and Mab 135
Antibodies on the RPMI 8866 Cell Line


     c-        c+          Mab 25          Mab 135
     8         82        29  (71.6)      56.3  (34.7)


Values are given as percentages.  Unbracketed figure
gives percentage of positive cells and bracketed figure
gives percentage inhibition.
```

Key:

c-: negative control obtained by incubation of RPMI 8866 cells with polyclonal anti-IgE antibody (1/1000) and anti-rabbit Ig-coated microspheres.

c+: positive control obtained by incubation of RPMI 8866 cells with 1 $\mu$g IgE, polyclonal anti-IgE antibody and anti-rabbit Ig-coated microspheres.

Hybridomas 9P25 and 9P135 D6/5 have been deposited at the Institute Pasteur, Paris, France under the numbers I 568 and I 549 respectively.

### 2. Binding pattern to cell lines

a) Technical aspects

Cells lines are maintained in the standard culture medium RPMI 1640 described above.

Cytofluorographic analysis of monoclonal antibodies binding to cell lines was performed by indirect immunofluorescense with fluorescein-conjugated goat anti-mouse immunoglobulins (G/M FITC) (Grub Laboratories, Vienna, Austria) utilizing a FACS 440. In brief, $5 \times 10^5$ cells were treated with 0.15 ml of the Mab (diluted or undiluted), incubated at 4°C for 30 minutes, and washed twice. The cells were then reacted with 0.15 ml of a 1:40 dilution G/M FITC at 4°C for 30 minutes, centrifuged, and washed three times. Cells were then analyzed on the FACS 440, and the intensity of fluorescence per cell was recorded on a pulse-height analyzer. Background staining was obtained by substituting a 0.15 ml aliquot of 1:500 ascites from a mouse intraperitoneally injected with a non-producing hybrid clone.

b) Mab 25 (Table II)

It does not bind to Molt 4 cells (a T cell leukemic line); Daudi (a Burkitt lymphoma) and a wide number of different $IL_2$-dependent human T cell clones (although some of them are found weakly positive). It binds to EBV infected lymphoblastoid B cell lines. It binds weakly to the myelomonocytic cell line U937. The binding of this antibody to the cell lines correlates very well with their $Fc_\varepsilon R_{II}$ expression.

## TABLE II

### Phenotypic expression of Mabs against Fc RII on different human cell lines $\varepsilon$

| | *$Fc_\varepsilon R_{II}$ | Mab 25 | Mab 135 | **Mab Anti-DR | Control |
|---|---|---|---|---|---|
| U 937 | ± | 16.4 | 13.4 | 95.0 | 1.4 |
| Molt 4 | − | 0.8 | 0.6 | 1.2 | 0.2 |
| DAUDI | − | 2.1 | 96.4 | 99.2 | 0.9 |
| RPMI 8866 | + | 94.1 | 91.8 | 99.8 | 1.1 |
| MO 14 | + | 97.8 | 98.0 | 98.3 | 4.8 |
| BME | + | 97.9 | 89.5 | 99.5 | 10.4 |

*Determined by microsphere (bead) assay described above.
**Clone L243 obtained from Becton Dickinson. DR antigens are histocompatibility antigens of Class II.

c) Mab 135 (Table II).

It does not bind to Molt 4 cells but does bind to Daudi cells as well as to all the $IL_2$-dependent $Fc_\varepsilon R_{II}$-negative T cell clones that have been tested. It also binds to all the EBV infected lymphoblastoid cell lines which have been tested and to the EBV negative B cell lines MO14 and BME. It binds weakly to U937.

In summary, Mab 135 is able to prevent binding of IgE to $Fc_\varepsilon R_{II}$ positive cells; and it also recognizes antigenic determinants which are present on $Fc_\varepsilon R_{II}$ negative cells.

3. Binding pattern to human blood, tonsil and spleen cells

a) Technical aspects

Blood, tonsil and spleen mononuclear cells are isolated according to standard procedures. Tonsils were obtained at tonsillectomy from children with chronic tonsillitis and spleens were obtained from operative speci-

7

mens of trauma patients. Spleens and tonsils were made into single cell suspensions (in PBS) by being pressed through wire mesh. These cell suspensions as well as diluted blood (in PBS) were next layered over Ficoll-Hypaque and centrifuged (following the technique of Boyum, Scand. J. Clin. Lab. Invest. 21 (supp. 97): 77, 1968). Mononuclear cells were harvested from the Ficoll interface and washed with PBS.

Cytofluorographic analysis of monoclonal antibodies with all cell populations was performed by indirect immunofluorescence as described earlier. Double fluorescence analysis was also performed in order to determine to which cell subpopulation the Mabs bind. The experimental protocol is described in Table III.

Data are summarized in Tables IV, V and VI.

## TABLE III

### PROCEDURE FOR DOUBLE STAINING CELLS WITH TWO MONOCLONAL ANTIBODIES

1. Adjust cells at $10^7$/ml in PBS, 1% BSA, 0.01% Azide.

2. Add the first monoclonal antibody at the appropriate concentration to 40 µl of the cell suspension (in microtiter tray wells).

3. Incubate 15 minutes at 4°C.

4. Wash twice with PBS, 1% BSA, 0.01% Azide.

5. Resuspend cells in 100 µl of a 1/150 dilution in PBS, FITC conjugated $F(ab')_2$ fragments goat anti-mouse immunoglobulins (Grub, Vienna, Austria).

6. Incubate 15 to 30 minutes at 4°C.

7. Wash twice in PBS-BSA-Azide.

8. Resuspend cells in 100 µl PBS-BSA containing 1% mouse serum.

9. Incubate 20 minutes at 4°C and wash twice.

10. Resuspend cells in 40 µl of PBS-BSA.

11. Add the second monoclonal antibody conjugated with biotin.

12. Incubate 15 minutes at 4°C.

13.   Wash twice with PBS-BSA-Azide.   .

14.   Resuspend cells in 40 µl PBS-BSA-Azide and add Avidin conjugated to Phycoerythrin (Becton Dickinson).

15.   Wash twice and resuspend cells in 250 µl PBS-BSA.

16.   Analyze with FACS.

In Tables IV - VI:
- indicates negatively reacting cells (Mab unbound);
+ indicates positively reacting cells (Mab fixed).
Leu 1 is a Mab commercialized by Becton Dickinson which specifically binds to T cells.
B1 is a Mab commercialized by Coulter Immunology which specifically binds to B cells.
Anti-DR is the Mab defined in Table II.
Leu M3 is a Mab commercialized by Becton Dickinson which specifically binds to monocytes.

## TABLE IV

### PERCENTAGE OF SPLEEN CELLS BOUND BY MABS

| SPLEEN* | | | Mab 25 | | Mab 135 | |
|---|---|---|---|---|---|---|
| | | | − | + | − | + |
| TOTAL CELLS | | | 4.8 | | 52.2 | |
| Leu 1 | (31.6) | − | 67.3 | 4.8 | 15.3 | 48.3 |
| | | + | 27.9 | <1 | 36.4 | <1 |
| B1 | (54.8) | − | 39.2 | <1 | 47.4 | 10.1 |
| | | + | 56.4 | 4.4 | 3.0 | 39.5 |
| Anti-DR | (61.3) | − | 36.5 | <1 | 40.7 | <1 |
| | | + | 58.3 | 5.0 | 1.7 | 57.6 |
| Leu M3 | (5.6) | − | 90.0 | 4.5 | 47.6 | 47.8 |
| | | + | 4.4 | 1.1 | 1.3 | 3.3 |

* Control:non-related Mab: 0.7

## TABLE V

### PERCENTAGE OF TONSIL CELLS BOUND BY MABS

| TONSILS* | | | Mab 25 | | Mab 135 | |
|---|---|---|---|---|---|---|
| | | | − | + | − | + |
| TOTAL CELLS | | | 9.4 | | 33.2 | |
| Leu 1 | (40.3) | − | 53.4 | 9.4 | 26.2 | 27.6 |
| | | + | 37.2 | <1 | 46.2 | <1 |
| B1 | (41.6) | − | 50.1 | 4.4 | 60.4 | 9.7 |
| | | + | 39.2 | 6.3 | 3.1 | 26.8 |
| Anti-DR | (43.8) | − | 50.7 | <1 | 62.3 | <1 |
| | | + | 41.8 | 7.5 | 2.5 | 35.2 |
| Leu M3 | (2.0) | − | 90.5 | 7.6 | 65.1 | 33.9 |
| | | + | <1 | 1.9 | <1 | 1.0 |

\*   Control:non-related Mab:  0.9

## TABLE VI

### PERCENTAGE OF PERIPHERAL BLOOD MONONUCLEAR CELLS BOUND BY MABS

| PBL* | | | Mab 25 | | Mab 135 | |
|---|---|---|---|---|---|---|
| | | | − | + | − | + |
| TOTAL CELLS | | | 4.0 | | 7.3 | |
| Leu 1 | (67.5) | − | 28.6 | 5.7 | 28.1 | 7.3 |
| | | + | 65.7 | <1 | 64.6 | <1 |
| B1 | (8.1) | − | 90.3 | <1 | 87.6 | 5.1 |
| | | + | 6.6 | 3.1 | 1.6 | 5.7 |
| Anti-DR | (20.1) | − | .76.5 | <1 | 78.5 | <1 |
| | | + | 19.9 | 3.6 | 13.4 | 8.1 |
| Leu M3 | (12.0) | − | 84.9 | 4.3 | 80.1 | 8.2 |
| | | + | 9.7 | 1.1 | 9.5 | 2.2 |

* Control:non-related Mab:  0.7

b) Mab 25

This antibody binds to approximately 4% of blood mononuclear cells, 4.8% spleen mononuclear cells and 9.4% tonsil mononuclear cells. It does not bind to T cells (Leu 1 positive). It preferentially binds to B cells (B1 positive) but not all of them. It also stains some non-T non-B lymphocytes, some Leu M3$^+$ cells (specific for monocytes) and some Leu M3$^-$ cells.

c) Mab 135

This antibody binds to approximately 7.3% blood mononuclear cells, 52.2% spleen mononuclear cells and 33.2% tonsil mononuclear cells. It does not bind to T cells (Leu 1 positive). It binds to almost all B cells (BI positive) and to a fraction of the monocytes (Leu M3 positive). It binds to almost all the DR positive spleen and tonsil mononuclear cells but only to a fraction of blood DR positive cells.

4. Binding pattern to activated PBL

a) Technical aspects

Isolated peripheral blood mononuclear cells are resuspended at $5 \times 10^5$ cells/ml into culture medium (RPMI 1640 + glutamine 2 mM + penicillin (100 U/ml) + streptomycin (100 µg/ml) + 10% Fetal Calf Serum) in the presence of the following mitogens:

Phytohemagglutinin 1% (PHA) (GIBCO, Grand Island); Concanavalin A 10 µg/ml (Con A) (Pharmacia, Uppsala, Sweden); Staphylococcus aureus strain Cowan I, 0.01% (SAC) (Pansorbin, Calbiochem, La Jolla, Ca.); Pokeweed Mitogen, 1/100 dilution (PWM) (GIBCO, Grand Island, NY). Cells were harvested five days later, washed three times and stained with the Mabs as described earlier. Data are given in Table VII.

## TABLE VII

### BINDING OF MABS 25 AND 135 TO PBL
### (PERIPHERAL BLOOD MONONUCLEAR CELLS)
### STIMULATED FOR 5 DAYS WITH VARIOUS MITOGENS

|        | CONTROLS | MAB 25 | MAB 135 |         |
|--------|----------|--------|---------|---------|
| CON A  | 3.6%     | 2.3%   | 42.8%   |         |
| PHA    | 5.9%     | 5.9%   | 49.3%   | EXPT 1  |
| PWM    | 1.1%     | 4.2%   | 28.8%   |         |
| COWAN  | 7.7%     | 21.0%  | 23.8%   |         |
| CON A  | ND       | ND     | ND      |         |
| PHA    | 4.7%     | 16.4%  | 52.2%   | EXPT 2  |
| PWM    | 0.8%     | 1.4%   | 42.5%   |         |
| COWAN  | 4.1%     | 3.2%   | 20.5%   |         |

b) Mab 25

Its expression is only slightly or very slightly increased upon mitogenic activation.

c) Mab 135

The percentage of cells reacting with Mab 135 is increased upon mitogenic stimulation.

## 5. Immunoprecipitation analysis

a) Technical aspects

a1) Labelling of cells:

(50 to 100) x $10^6$ cells are first washed twice with PBS and then resuspended in 2 ml PBS. To this suspension the following reagents are added: 0.5-1 mCi Na$^{125}$I (Amersham,) 100 µl lactoperoxidase at 5 mg per ml (Calbiochem) and 50 µl $H_2O_2$ (0.03% in water). The suspension is agitated for 3 minutes at room temperature. Another 100 µl lactoperoxidase and 50 µl $H_2O_2$ are added. The suspension is again agitated for 3 minutes at room temperature. This step is repeated once again and the cells are finally washed with PBS containing 5mM KI. The labelled cells are then resuspended at 5 x $10^6$ per ml in PBS containing 0.5% Nonidet P-40, 1mM phenylmethylsulfonyl fluoride (PMSF), 0.01 M benzamidine hydrochloride, 0.05M aminocaproic acid, 20 mM iodoacetamide, 10 µg/ml leupeptin, 1 µg/ml pepstatin and 100 µg/ml soybean trypsin inhibitor, and are incubated at 0°C for 20 minutes. The cells are centrifuges at 3000X g for 20 minutes and the supernatant is stored at -70°C. The lysate is clarified by centrifugation at 100,000X g for 30 minutes.

a2) Immunoprecipitation of membrane antigens:

The lysates are first precleared overnight with an ascite containing a non-related monoclonal antibody. Then, a rabbit anti-mouse immunoglobulin coupled to formalinized Staphylococcus aureus strain Cowan is added. After centrifugation the lysate is ready for the immunoprecipitation, which is performed as in the preclearing step except that the non-related Mab is replaced by the antibody to be assayed.

b) Mab 25:

It specifically precipitates a 42-44 Kd molecule which corresponds to the established data for $Fc_\varepsilon R_{II}$ using affinity chromatography on immobilized IgE.

c) Mab 135:

It immunoprecipitates three different molecular entities: a 44-46 Kd molecule (which may be different from that precipitated by Mab 25) and also molecules of about 35 Kd and about 30 Kd.

## 6) Determination of the affinity (Ka) of Mab 25

The affinity of Mab 25 for its specific cell surface antigen was performed according to Trucco and de Petris (Immunological Methods, Vol III, Ed: I. Lefkovits, B. Pernis, Academic press, 1981, pp. 1-26).

a. Technical aspect

a1. Iodination of Mab 25

50 µg of Mab 25 in 100 µl of PBS are mixed with 1 mCi Na$^{125}$I (5 µl) and chloramine T (5 µl at 0.8 mg per ml in PBS). After a 10 minutes' incubation period at room temperature under gentle shaking, 5 µl sodium bisulphite (1 mg/ml in PBS) and 250 µl of ovalbumin (5% in PBS) are added to stop the reaction. The solution is passed over a PD10 column (Pharmacia, Sweden) pre-equilibrated with PBS containing 0.1% Tween 20 (Merck, Germany) and 1% bovine serum albumin. This step permits recovery of the iodinated antibody without free iodine.

## a2. Determination of the affinity and of the number of receptors

Increasing concentrations (0.1 µg to 4µg) of the iodinated Mab 25 are added to $10^6$ RPMI 8866 cells in a final volume of 1 ml of RPMI 1640 medium containing 2% BSA. For antibody concentrations of 0.3, 0.5 and 1 µg/ml, additional tubes are prepared in which non-labelled Mab 25 is added in a 500-fold excess in order to determine non-specific antibody binding. Binding lasts 3 hours at 4°C. Cells are finally washed three times and the radioactivity in the final cell pellet is determined in a gamma counter. The data are finally plotted using the standard Scatchard analysis.

## b. Affinity and number of receptors of Mab 25

The affinity of Mab 25 for the RPMI 8866 $Fc_\varepsilon R_{II}$ is $1.2 \times 10^8 M^{-1}$ and 435,000 receptors are identified.

## Kit for detection of cellular $Fc_\varepsilon R_{II}$

I) Detection of $Fc_\varepsilon R_{II}$ on peripheral blood cells.

Conventional IgE rosette assays suggest that atopic patients display increased expression of $Fc_\varepsilon R_{II}$ on their blood mononuclear cells. Mab 25 permits a much more accurate determination of $Fc_\varepsilon R_{II}$ expression and is useful in this type of clinical investigation. Determining the expression of $Fc_\varepsilon R_{II}$ on blood mononuclear cells is useful in a number of clinical situations related to atopy. The Mab 25 can be made available in a lyophilized form either unlabelled in itself but accompanied by an appropriately labelled second stage antimouse Ig antibody or labelled with either FITC or biotin. To detect biotin as label, FITC- or phycoerythrin-labelled avidin can be used.

II) Detection of $Fc_\varepsilon R_{II}$ on human T cell clones.

It has been demonstrated in rodents that $Fc_\varepsilon R_{II}$-positive T cells play a major role in the regulation of IgE synthesis. Mab 25 is presently used to establish human $Fc_\varepsilon R_{II}$-positive $IL_2$-dependent human T cell clones. Such clones should prove highly valuable tools to elucidate the regulation of IgE production in humans.

## Kit for detection of soluble $Fc_\varepsilon R_{II}$ by competition assay

Mab 25 is coupled to beads or wells of microtiter plates. Samples to be assayed for the presence of soluble $Fc_\varepsilon R_{II}$ or a standard preparation of $Fc_\varepsilon R_{II}$ are first incubated with a known amount of labelled soluble $Fc_\varepsilon R_{II}$. After incubation they are transferred to and allowed to react with the insolubilized antibody on the beads or in the wells. The labelled soluble $Fc_\varepsilon R_{II}$ can be either i) a $^{125}I$ iodinated recombinant $Fc_\varepsilon R_{II}$; ii) an artificial fusion product between $Fc_\varepsilon R_{II}$ and an enzyme e.g. E. coli β-galactosidase; or iii) a biotinylated $Fc_\varepsilon R_{II}$. In case i) the reaction will be monitored through gamma counting; in case ii) the reaction will be monitored through optical measurement of degradation substrates specific for the enzyme (e.g. the reaction will be monitored by β galactosidase); in case iii) optical measurement of degradation substrates specific for the enzyme which was coupled to the biotin-specific avidin. In any case the presence of soluble $Fc_\varepsilon R_{II}$ in the sample to be assayed will induce a decrease in the observed signal.

## Claims

1. Monoclonal antibodies inhibiting binding of soluble IgE to human lymphocytes, wherein said monoclonal antibodies are produced by a hybridoma cell line selected from the group consisting of the hybridoma cell lines deposited with the Institut Pasteur and having the deposit accession numbers I-548 and I-549.

2. Monoclonal antibodies to the human lymphocyte IgE receptors, wherein said monoclonal antibodies are produced by a hybridoma cell line selected from the group consisting of the hybridoma cell lines deposited with the Institut Pasteur and having the accession numbers I-548 and I-549.

3. A hybridoma cell line which is deposited with the Institut Pasteur under the accession number I-548.

4. A hybridoma cell line which is deposited with the Institut Pasteur under the accession number I-549.

5. Use of a monoclonal antibody according to claim 1 or 2 to assay cells bearing the human $Fc_\varepsilon R_{II}$ receptors.

6. Use of a monoclonal antibody produced by the hybridoma cell line according to claim 3, to immunoprecipitate a 42-44 Kd menbrane molecule which is the $Fc_\varepsilon R_{II}$.

7. Use of a monoclonal antibody produced by the hybridoma cell line according to claim 4, to immunoprecipitate three membrane molecules with molecular weights in the regions of 44-46, 35 and 30 Kd.

8. A method for detecting soluble $Fc_\varepsilon R_{II}$ in a sample comprising the steps of:
- coupling a monoclonal antibody produced by a hybridoma cell line according to claim 3, to beads or wells,
- incubating the sample to be assayed for the presence of soluble $Fc_\varepsilon R_{II}$ with a known amount of labelled soluble $Fc_\varepsilon R_{II}$,
- reacting said incubated sample with the insolubilized antibody on the beads or wells, and
- detecting the presence of soluble $Fc_\varepsilon R_{II}$ in the sample by a decrease of the signal emitted by the labelled soluble $Fc_\varepsilon R_{II}$ which immunoreacts with the monoclonal antibody.

9. A kit for detecting cellular $Fc_\varepsilon R_{II}$ on peripheral blood cells comprising a monoclonal antibody produced by a hybridoma cell line according to claim 3, said monoclonal antibody being either itself labelled with FITC or biotin or unlabelled and accompanied by an appropriately labelled antimouse Ig antibody.

10. A kit for establishing human $Fc_\varepsilon R_{II}$-positive $Il_2$-dependent human T cell clones, comprising a monoclonal antibody produced by a hybridoma cell line according to claim 3.

## Patentansprüche

1. Monoklonale Antikörper, die die Bindung von löslichem IgE an humane Lymphozyten inhibieren, wobei die gennanten monoklonalen Antikörper von einer Hybridom-Zellinie, ausgewählt aus der Gruppe von Hybridom-Zellinien, die unter den Eingangsnummern I-548 und I-549 am Institut Pasteur hinterlegt sind, produziert werden.

2. Monoklonale Antikörper gegen humane Lymphozyt-IgE-Rezeptoren, wobei die gennanten monoklonalen Antikörper von einer Hybridom-Zellinie, ausgewählt aus der Gruppe von Hybridom-Zellinien, die unter den Eingangsnummern I-548 und I-549 am Institut Pasteur hinterlegt sind, produziert werden.

3. Hybridom-Zellinie, welche unter der Eingangsnummer I-548 am Institut Pasteur hinterlegt ist.

4. Hybridom-Zellinie, welche unter der Eingangsnummer I-549 am Institut Pasteur hinterlegt ist.

5. Verwendung eines monoklonalen Antikörpers nach Anspruch 1 oder 2, um Zellen mit humanen $Fc_\varepsilon R_{II}$ Rezeptoren zu untersuchen.

6. Verwendung eines monoklonalen Antikörpers, produziert von der Hybridom-Zellinie nach Anspruch 3, zur Immunfällung eines 42-44 Kd Membranmoleküls, welches $Fc_\varepsilon R_{II}$ ist.

7. Verwendung eines monoklonalen Antikörpers, produziert von der Hybridom-Zellinie nach Anspruch 4, zur Immunfällung von drei Membranmolekülen mit Molekulargewichten im Bereich 44-46, 35 und 30 Kd.

8. Verfahren zur Detektion von löslichem $Fc_\varepsilon R_{II}$ in einer Probe, bestehend aus folgenden Schritten:
- Kopplung eines monoklonalen Antikörpers, der von einer Hybridom-Zellinie nach Anspruch 3 produziert wurde, an Kügelchen oder Vertiefungen,
- Inkubation der Probe, die auf Anwesenheit von löslichem $Fc_\varepsilon R_{II}$ untersucht werden soll, mit einer bekannten Menge markiertem löslichen $Fc_\varepsilon R_{II}$,
- Reaktion der genannten inkubierten Probe mit dem Antikörper, der auf Kügelchen oder Vertiefungen unlöslich gemacht worden ist, und
- Detektion der Anwesenheit von löslichem $Fc_\varepsilon R_{II}$ in der Probe durch Abnahme des Signals, das vom markierten löslichen $Fc_\varepsilon R_{II}$, das mit dem monoklonalen Antikörper immunreagiert, emittiert wird.

9. Testkombination zur Bestimmung von zellulärem $Fc_\varepsilon R_{II}$ auf periphären Blutzellen, die einen monoklonalen Antikörper, produziert von einer Hybridom-Zellinie nach Anspruch 3, umfaßt, wobei der genannte monoklonale Antikörper entweder selbst mit FITC oder Biotin markiert oder unmarkiert und mit einem entsprechend markierten Antimaus-Ig-Antikörper verbunden sein kann.

10. Testkombination zur Etablierung von humanen $Fc_\varepsilon R_{II}$-positiven $IL_2$-abhängigen humanen T-Zellklonen, die einen monoklonalen Antikörper, produziert von einer Hybridom-Zellinie nach Anspruch 3 umfaßt.

## Revendications

1. Anticorps monoclonaux inhibant la liaison de l'IgE soluble aux lymphocytes humains, où les anticorps monoclonaux précités sont produits par une lignée de cellules d'hybridomes sélectionnée dans le groupe consistant en lignées de cellules d'hybridomes déposées à l'Institut Pasteur et ayant les numéros de dépôts I-548 et I-549.

2. Anticorps monoclonaux contre des récepteurs d'IgE de lymphocyte humain, où les anticorps monoclonaux précités sont produits par une lignée de cellules d'hybridomes sélectionnée dans le groupe consistant en lignées de cellules d'hybridomes déposées à l'Institut Pasteur et ayant les numéros de dépôts I-548 et I-549.

3. Lignée de cellules d'hybridomes qui est déposée à l'Institut Pasteur sous le numéro de dépôt I-548.

4. Lignée de cellules d'hybridomes qui est déposée à l'Institut Pasteur sous le numéro de dépôt I-549.

5. Utilisation d'un anticorps monoclonal selon la revendication 1 ou 2 pour tester des cellules portant des récepteurs humains $Fc_\varepsilon R_{II}$.

6. Utilisation d'un anticorps monoclonal produit par la lignée de cellules d'hybridomes selon la revendication 3, pour immunoprécipiter une molécule de membrane de 42-44 Kd qui est le $Fc_\varepsilon R_{II}$.

7. Utilisation d'un anticorps monoclonal produit par la lignée de cellules d'hybridomes selon la revendication 4, pour immunoprécipiter trois molécules de membrane ayant des poids moléculaires dans les régions de 44-46, 35 et 30 Kd.

8. Procédé pour détecter un $Fc_\varepsilon R_{II}$ soluble dans un échantillon comprenant les étapes de :
– coupler un anticorps monoclonal produit par une lignée de cellules d'hybridomes selon la revendication 3, à des billes ou des puits,
– incuber l'échantillon destiné à être testé pour la présence de $Fc_\varepsilon R_{II}$ soluble avec une quantité connue de $Fc_\varepsilon R_{II}$ soluble marqué,
– faire réagir l'échantillon incubé précité avec l'anticorps insolubilisé sur les billes ou les puits, et
– détecter la présence de $Fc_\varepsilon R_{II}$ soluble dans l'échantillon par une diminution du signal émis par le $Fc_\varepsilon R_{II}$ soluble marqué qui immunoréagit avec l'anticorps monoclonal.

9. Kit pour détecter $Fc_\varepsilon R_{II}$ cellulaire sur des cellules de sang périphérique, comprenant un anticorps monoclonal produit par une lignée de cellules d'hybridomes selon la revendication 3, l'anticorps monoclonal précité étant soit lui-même marqué avec le FITC ou la biotine, soit non marqué et accompagné d'un anticorps Ig antisouris marqué de manière appropriée.

10. Kit pour établir des clones de cellules T humaines dépendantes d'$IL_2$ positives à $Fc_\varepsilon R_{II}$ humain, comprenant un anticorps monoclonal produit par une lignée de cellules d'hybridomes selon la revendication 3.